# DEMANDE DE BREVET EUROPEEN

(11) **EP 0 880 943 A1**
(43) Date de publication de la demande: **02.12.1998**
(21) Numéro de dépôt: 98401307.8
(22) Date de dépôt: 29.05.1998
(51) Int. Cl.: A61B 17/88

(54) **Outil de vissage d'une vis à deux parties filetées séparées par une partie intermédiaire de vissage**

(30) Priorité: 30.05.1997 FR 9706696
(71) Demandeur: Société de Fabrication de Matériel Orthopédique en abrégé - SOFAMOR, 93290 Tremblay-en-France (FR)
(72) Inventeur: Bigand, Dominique, 60260 la Morlaye (FR); Clauze, Jacques, 64300 Baigts de Bearn (FR); Gournay, José, 77230 Dammartin-en-Goele (FR); Moreau, Jean-Charles, 60270 Gouvieux (FR); Saurat, Jean, 49240 Avrille (FR)
(74) Mandataire: Martin, Jean-Paul

(57) **Abrégé**

Cet outil comprend un tirant (5) à extrémité taraudée adaptée pour venir se visser sur l'une (3) des deux parties filetées d'ancrage osseux (2) et à filet mécanique (24), de la vis (1), une cage tubulaire (7) adaptée pour pouvoir coiffer une partie intermédiaire (4) de vissage et contenant des organes de roulement (9) pouvant être appliqués sur la surface de ladite partie intermédiaire (4); un tube (11) enveloppant la cage et le tirant, pourvu d'une poignée (12) d'entraînement en rotation et dont la paroi intérieure est profilée pour qu'une rotation du tube déplace radialement l'organe de roulement à travers la cage et le place en appui de serrage contre ladite partie intermédiaire (4) de la vis (1). Cet outil est applicable notamment en chirurgie orthopédique pour le vissage d'implants osseux. Il permet de supprimer des encoches sur la partie de vissage de la vis, donc des sources d'amorces de rupture. La mise en pression des galets constituant les organes de roulement est quasi immédiate par rotation du tube extérieur.

## Description

La présente invention a pour objet un outil de vissage d'une vis à deux tiges filetées séparées par une partie intermédiaire prévue pour recevoir des moyens de vissage.

Un tel outil trouve des applications chirurgicales très étendues, notamment en thoracoscopie et en ostéosynthèse rachidienne... pour la mise en place d'implants vissés.

On connaît des vis à deux tiges filetées de pas différents et dans le prolongement l'une de l'autre, séparées par une partie intermédiaire de préhension pour le vissage. Des vis de ce type sont décrites notamment dans le document EP-A-0 612 507. La partie intermédiaire entre les deux parties filetées peut être sphérique, conique ou hexagonale etc. Dans le cas où cette partie est sphérique ou conique, elle présente deux encoches latérales de préhension d'un outil de vissage. Ces encoches sont difficiles à trouver par le chirurgien pendant l'intervention chirurgicale, constituent des sources d'amorces de rupture, et nécessitent un poste d'usinage spécifique, ce qui entraîne un coût supplémentaire pour la fabrication de la vis.

Par ailleurs, les parties de préhension constituées par une empreinte hexagonale de vissage augmentent notablement l'encombrement de la vis, ce qui constitue un autre inconvénient.

L'invention a donc pour but de proposer un outil de vissage qui soit d'un emploi aisé par le chirurgien en cours d'intervention et présente une grande solidité.

Conformément à l'invention, l'outil de vissage comprend :
- un tirant à extrémité taraudée adaptée pour venir se visser sur l'une des deux parties filetées,
- une cage tubulaire disposée dans le prolongement de l'extrémité du tirant et en contact avec celle-ci, cette cage étant adaptée pour pouvoir coiffer ladite partie intermédiaire de vissage et étant percée d'au moins une ouverture contenant un organe de roulement pouvant être appliqué sur la surface de ladite partie intermédiaire par une sollicitation extérieure radiale,
- un tube enveloppant la cage et le tirant, pourvu de moyens d'entraînement en rotation autour de son axe longitudinal, dont la paroi intérieure est en contact avec ledit organe de roulement et profilée pour qu'une rotation du tube déplace radialement l'organe de roulement à travers la cage et le place en appui de serrage contre ladite partie intermédiaire de la vis.

Grâce à la structure ainsi réalisée, une faible rotation du tube extérieur autour de son axe plaque les organes de roulement sur la partie intermédiaire sphérique ou cylindrique de la vis, qui peut ensuite être vissée ou dévissée grâce à ce serrage.

Suivant un mode de réalisation de l'invention, l'outil comporte plusieurs organes de roulement constitués par des galets disposés avec jeu dans des ouvertures formées à intervalles angulaires réguliers dans la cage.

Ces galets peuvent être par exemple cylindriques et exercer une pression uniformément répartie sur la zone de vissage de l'implant.

Suivant un autre mode de réalisation de l'invention, l'outil de vissage comprend :
- un tirant à extrémité taraudée adaptée pour venir se visser sur l'une des deux parties filetées,
- un tube extérieur, contenant le tirant et dans une extrémité duquel la vis peut être partiellement introduite pour que l'une de ses parties filetées puisse être vissée dans le tirant, ce tube étant équipé de moyens d'entraînement en rotation autour de son axe longitudinal,
- une came transversale s'étendant diamétralement à travers ladite extrémité du tube adaptée pour recevoir la vis, et solidaire du tube en rotation,
- un élément de serrage de la vis, interposé dans le tube avec jeu entre la came et l'extrémité adjacente du tube, profilé pour pouvoir être déplacé en translation axiale et appliqué sur ladite partie intermédiaire de la vis par rotation du tube et de la came autour de l'axe longitudinal du tube.

Ainsi une rotation du tube extérieur autour de son axe fait également tourner la came autour du même axe, ce qui vient fermement appliquer l'élément précité sur la partie de vissage de la vis. Cet élément peut être constitué par exemple par une rondelle à profil intérieur complémentaire de celui de la partie de vissage sur laquelle elle vient s'appuyer.

D'autres particularités et avantages de l'invention apparaîtront au cours de la description qui va suivre, faite en référence aux dessins annexés qui en illustrent plusieurs modes de réalisation à titre d'exemples non limitatifs.

La figure 1 est une vue en coupe longitudinale d'une première forme de réalisation de l'outil de vissage selon l'invention ainsi que, en élévation longitudinale, d'une vis à deux parties filetées engagée dans une extrémité de l'outil.

La figure 2 est une vue analogue à la figure 1 d'une variante de réalisation de l'outil.

La figure 3 est une vue en coupe transversale suivant 3/3 de la figure 1 à échelle agrandie.

La figure 4 est une vue en élévation latérale à échelle agrandie de la cage de l'outil de vissage des figures 1 et 2.

La figure 5 est une vue de la cage de la figure 4 dans laquelle est partiellement introduite la vis à deux parties filetées selon la figure 2.

La figure 6 est une vue en coupe longitudinale d'une seconde forme de réalisation de l'outil de vissage selon l'invention.

La figure 7 est une vue en coupe longitudinale partielle à échelle agrandie de l'extrémité de l'outil de la figure 6 dans laquelle vient s'introduire la vis à visser ou à dévisser.

La figure 8 est une vue en coupe transversale suivant 8/8 de la figure 7.

La figure 9 est une vue en élévation à échelle agrandie de la came de l'outil des figures 6 à 8.

La figure 10 est une vue en perspective partielle à échelle agrandie de l'outil des figures 6 à 8, montrant sa came transversale en position neutre avant serrage de la vis.

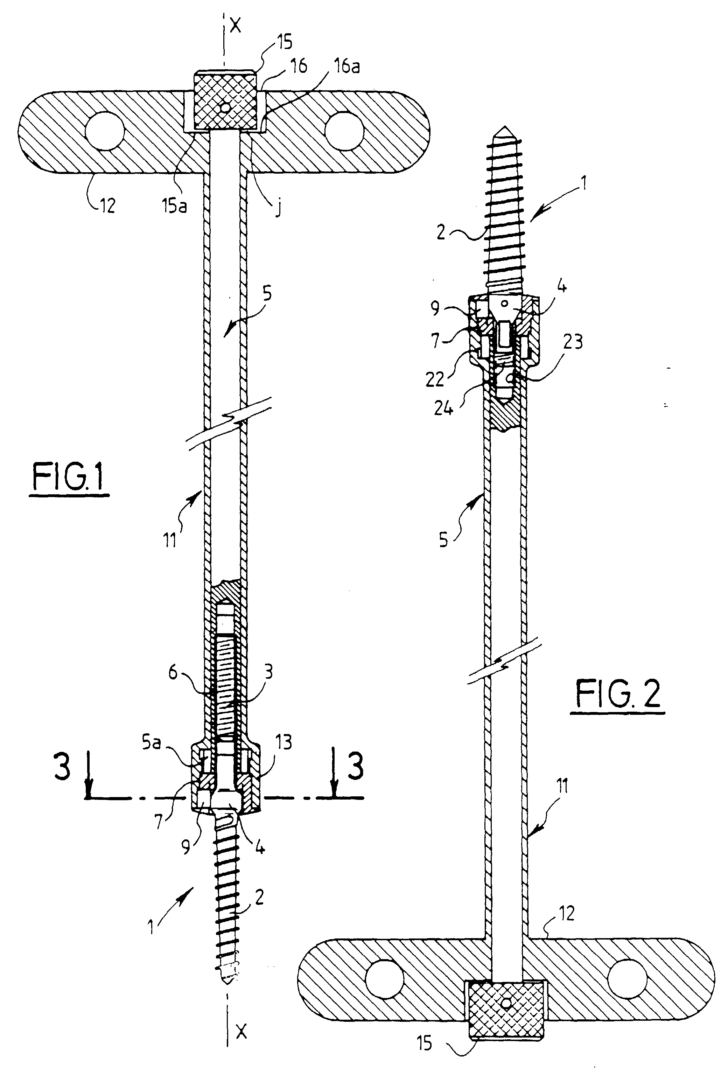

L'outil représenté aux figures 1 à 5 est destiné au vissage d'une vis 1 à deux tiges filetées 2, 3 situées dans le prolongement l'une de l'autre et séparées par une partie intermédiaire 4 prévue pour recevoir des moyens de vissage. Les tiges filetées 2 et 3 ont des pas différents, et la partie de préhension 4 pour le vissage peut être par exemple sphérique, conique, cylindrique ou présenter un contour hexagonal.

Des vis de ce type sont utilisées notamment en chirurgie orthopédique pour constituer des implants d'ancrage osseux par leur tige filetée 2.

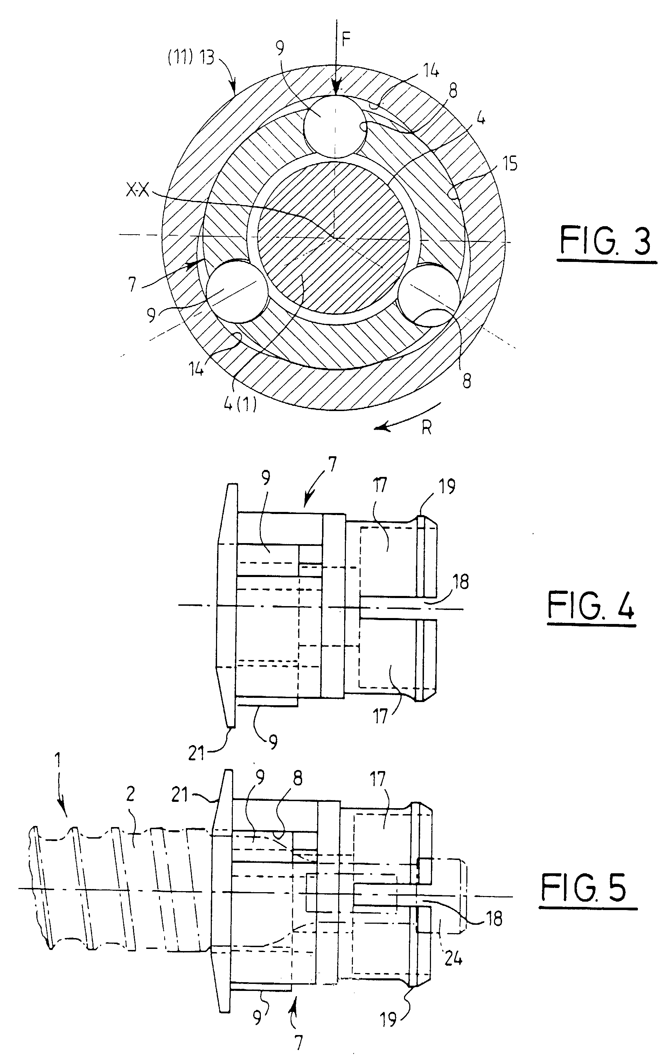

L'outil 1 comprend :
- un tirant 5 dont une extrémité 6 est taraudée et adaptée pour venir se visser sur la partie filetée de la tige 3, la tige filetée 2 étant destinée à être vissée, par exemple dans le pédicule d'une vertèbre pour une instrumentation d'ostéosynthèse rachidienne;
- une cage tubulaire 7 disposée dans le prolongement de l'extrémité 6 du tirant 5 et en contact avec celle-ci; la cage 7 est adaptée pour pouvoir coiffer la partie intermédiaire 4 de vissage de la vis 1, cette partie 4 ayant une surface cylindrique dans l'exemple représenté. La cage tubulaire 7 est percée de plusieurs ouvertures, à savoir trois ouvertures 8 dans l'exemple décrit, formées à intervalles angulaires égaux autour de la partie de vissage 4; ces ouvertures 8 contiennent chacune un organe de roulement 9 constitué par un galet, pouvant être appliqué sur la surface de la partie intermédiaire 4 par une sollicitation extérieure radiale F (figure 3).
- L'outil 1 comprend aussi un tube extérieur 11 enveloppant la cage 7 et le tirant 5. Ce tube extérieur 11 est muni de moyens d'entraînement en rotation autour de son axe longitudinal XX, constitués dans l'exemple représenté par une poignée manuelle transversale 12 formée à son extrémité opposée à celle qui reçoit la cage 7. La partie terminale 13 du tube 11, opposée à sa poignée 12, présente un diamètre sensiblement supérieur à celui du reste du tube 11, afin d'envelopper sans jeu la cage cylindrique 7. La paroi intérieure de la partie terminale 13 est profilée de manière appropriée pour qu'une rotation du tube 11 autour de son axe XX exerce sur les galets 9 des forces radiales F qui les déplacent en appui sur la partie de vissage 4 de la vis 1.

Dans l'exemple illustré à la figure 3, des empreintes circulaires 14, de rayon supérieur à celui du reste de la paroi intérieure 15 de la partie 13, sont ainsi formées en regard de chaque galet 9 symétriquement par rapport à celui-ci. On comprend donc qu'une rotation du tube extérieur 11 dans un sens ou dans l'autre autour de son axe XX déplace radialement les galets 9 montés avec jeu dans les ouvertures 8 de la cage 7, jusqu'à ce qu'ils soient appliqués sur la surface de la partie 4 avec une force dépendant du couple de rotation exercé sur la poignée 12.

Le tirant 5 s'étend à l'intérieur du tube extérieur 11, traverse la poignée 12 et se termine par un bouton manuel 15 de commande en rotation. Le bouton 15 présente une surface à aspérités, par exemple moletée, et est placé dans un logement 16 de la poignée 12, un jeu de fonctionnement j étant délimité entre le fond 16a du logement 16 et la face transversale 15a du bouton 15. Par ailleurs le tirant 5 est libre en translation axiale à l'intérieur du tube 11.

La cage tubulaire 7 est montée dans l'extrémité 13 du tube 11 de manière amovible, par exemple à l'aide de moyens de clipsage permettant une extraction aisée de la cage 7 de la partie terminale 13. Dans la réalisation décrite (figures 4 et 5), ces moyens de clipsage comprennent plusieurs languettes longitudinales flexibles 17, par exemple trois séparées par des fentes longitudinales 18 et qui présentent chacune un bossage annulaire terminal 19. A l'opposé des languettes 17, la cage 7 est pourvue d'une collerette transversale 21 adaptée pour venir s'appliquer sur le bord terminal de la partie 13 du tube 11, après introduction de la cage 7 dans celle-ci.

A la fin de cette introduction axiale de la cage 7, les bossages 19 viennent se loger élastiquement dans une gorge complémentaire 22 formée dans l'extrémité de la paroi intérieure de la partie terminale 13, qui retient ainsi en place la cage 7. Pour extraire celle-ci en cas de besoin, il suffit d'exercer à l'aide d'un outil approprié une traction sur la collerette 21.

En fonction de la longueur de la tige filetée 3 de la vis 1, qui peut varier selon le modèle de vis, l'alésage taraudé 6 peut avoir une longueur correspondante différente. Ainsi dans la réalisation de la figure 2, le tirant 5 comporte un alésage 23 de longueur très inférieure à celle de l'alésage 6, afin de recevoir une partie filetée correspondante 24, plus courte que la partie filetée 3, le reste de l'outil étant identique à l'outil de la figure 1.

Plus précisément les alésages 6, 23 constituent des trous taraudés aptes à recevoir les parties filetées des tiges 3, 24.

La mise en oeuvre de l'outil qui vient d'être décrit découle directement de la description qui en a été faite.

On choisit l'outil dont le tirant 5 présente un alésage 6 ou 23... de longueur adaptée à celle de la vis 1 que l'on veut utiliser. On introduit la tige filetée 3 ou 24 dans l'alésage 6 ou 23 et on visse son extrémité dans le trou terminal 6a ou 23a. En fin de vissage, la partie intermédiaire de vissage 4 se trouve placée dans la cage 7, en regard radialement des galets 9. la tige filetée 2 faisant saillie à l'extérieur de la cage 7.

En fin de vissage du tirant 5 sur la tige filetée 3 ou 23 l'extrémité du tirant vient en contact de butée avec la cage 7, et le jeu précité j subsiste entre le bouton 15 et le fond 16a du logement 16. De ce fait, lorsqu'on fait tourner la poignée 12 dans un sens ou dans l'autre, afin de visser ou dévisser la vis 1, les galets 9 sont plaqués sur la surface de la partie 4 par la rotation des empreintes 14, et le vissage ou le dévissage s'exerce avec persistance du jeu j entre le bouton 15 et le fond du logement 16. Grâce à ce jeu, le bouton 15 ne peut gêner la rotation de la poignée 12, et par conséquent l'effort de vissage ou de dévissage exercé par l'opérateur.

Le jeu de fonctionnement est important pour une utilisation satisfaisante de l'outil. En effet en son absence, en fin de vissage, le bouton 15 du tirant 5 viendrait se bloquer dans la poignée 12, de sorte qu'il faudrait fournir un effort considérable pour débloquer l'outil 11 après vissage de la vis 1, ou au contraire pour le débloquer.

Il suffit d'une rotation de très faible amplitude du tube 11 pour serrer fortement les galets 9 sur la partie 4, ce qui constitue un avantage important de l'outil selon l'invention.

Un autre avantage de cet outil résulte de la présence de la cage 7. En effet en l'absence de celle-ci, l'extrémité du tube extérieur 11 peut rencontrer des zones molles ou au contraire des points durs, par exemple en chirurgie des tissus mous et des points durs osseux. Ceux-ci provoqueraient alors une remontée du tube, et donc l'arrêt du vissage ou du dévissage. La cage tubulaire intercalaire 7 empêche une telle remontée et permet donc de maintenir l'effort de vissage dans les cas ci-dessus. Ceci est particulièrement important, en raison de la faible longueur de guidage entre les galets 9 et la portée d'appui de la partie 4, qui peut être en effet par exemple sphérique.

Ainsi la cage tubulaire 7 garantit la continuité et l'efficacité de l'opération de vissage-dévissage.

En outre le fait que cette cage 7 soit amovible constitue un autre avantage, car il permet un démontage aisé de l'outil 11, après par exemple une intervention chirurgicale, à des fins de nettoyage.

Par ailleurs, en fin de vissage du tirant 5 sur la partie filetée 3, 24, l'extrémité 5a du tirant 5 vient en butée sur la cage 7 (figures 1 et 2). Cette mise en butée entraîne la cage 7 en rotation et met donc les galets 9 en pression sur le tube extérieur 11, ces galets ainsi que la cage 7 ayant donc été déplacés en rotation dans le sens horaire R (figure 3). Il en résulte que l'entraînement en rotation du tube 11 par la poignée 12 s'effectue ensuite sans temps de mise en charge préalable des galets, ce qui provoque avantageusement le serrage immédiat de ceux-ci sur la vis 1.

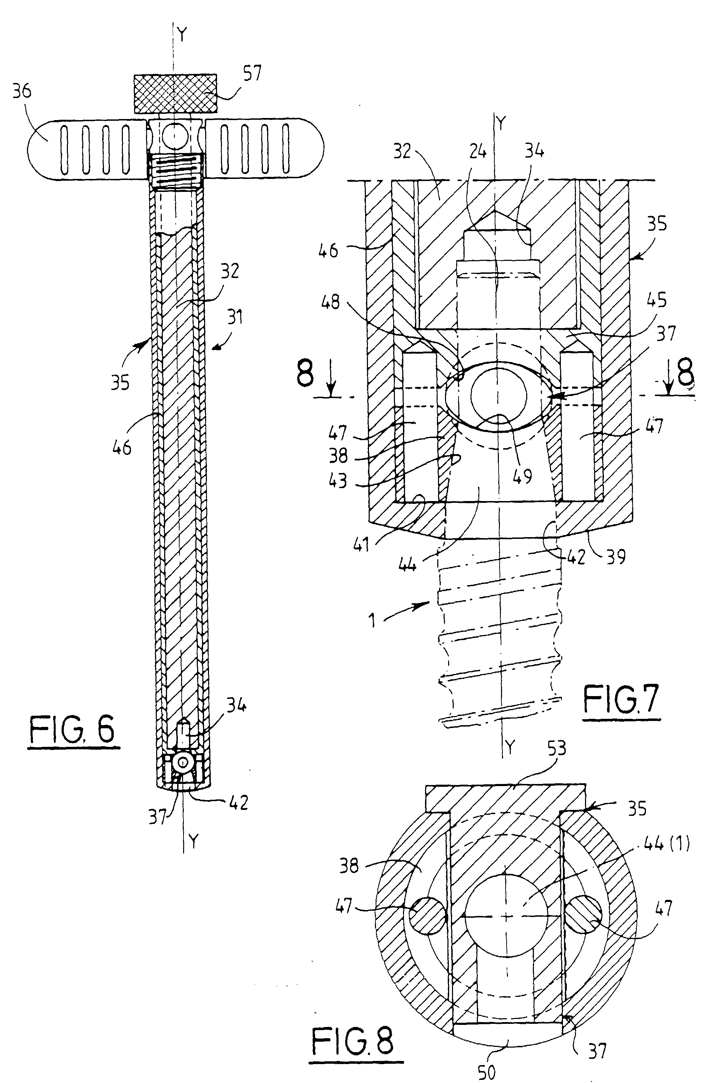

On décrira maintenant un second mode de réalisation de l'outil de vissage selon l'invention en se référant aux figures 6 à 10.

L'outil 31 comprend les éléments suivants :
- un tirant 32 d'axe longitudinal YY dont une extrémité présente un trou axial taraudé 34 adapté pour venir se visser sur l'une 24 des deux parties filetées 3, 24 de la vis;
- un tube extérieur 35 d'axe longitudinal YY, contenant le tirant 32 et dans une extrémité duquel la vis 1 peut être partiellement introduite pour que sa tige filetée 24 (3a) puisse être vissée dans le trou taraudé 34; le tube 35 est équipé de moyens d'entraînement en rotation autour de son axe longitudinal YY, à savoir une poignée terminale 36 prévue à l'extrémité opposée au trou 34.
- une came transversale 37 s'étendant diamétralement à travers l'extrémité du tube 35 adaptée pour recevoir la vis 1, et solidaire du tube 35 en rotation.
- Un élément 38 interposé dans le tube 35 avec jeu entre la came 37 et l'extrémité adjacente 39 du tube 35, et plus précisément la paroi intérieure 41 de l'extrémité 39. Cette dernière est percée d'un trou central 42 permettant l'introduction d'une vis 1 dans l'élément annulaire 38, constitué par exemple par une rondelle à surface intérieure conique 43 adaptée pour venir coiffer une surface conique complémentaire de la partie de vissage 44 de la vis 1.

Dans la réalisation décrite, l'outil 31 comporte un second élément 45 interposé entre la came 37 et le tirant 32. Le second élément 45 constitue l'extrémité d'un tube intercalaire 46 disposé entre le tirant 32 et le tube extérieur 35. La partie terminale 45 du tube 46 est reliée à la rondelle 38 par des moyens laissant à la rondelle 38 un degré de liberté en translation axiale dans le tube 35. Dans l'exemple illustré à la figure 7, ces moyens sont constitués par deux goujons longitudinaux 47 de liaison, logés dans des alésages correspondants de l'extrémité 45 du tube 46 et de la rondelle 43. Les faces en vis-à-vis 48, 49 des parties 45 et 38, situées de part et d'autre de la came 37, sont profilées pour constituer des surfaces complémentaires de la surface de la came 37.

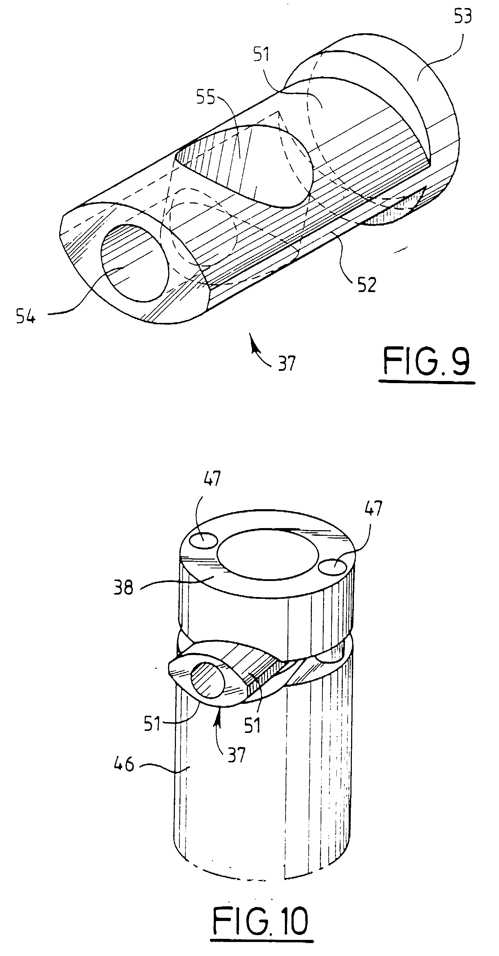

Cette dernière se présentant dans l'exemple de réalisation décrit sous forme d'une barrette de section ovale ou sensiblement elliptique ou ellipsoïde 51 (figures 9 et 10), les surfaces 48, 49, situées entre les goujons 47, ont un profil conjugué des deux côtés de la came 37.

Entre les deux faces ellipsoïdes 51, sont prévus des méplats longitudinaux 52. A l'une de ses extrémités la came 37 se prolonge par un disque 53, tandis que son extrémité opposée est percée d'un trou central 54 adapté pour recevoir un goujon (non représenté) permettant de solidariser la came 37 avec le tube extérieur 35. Les extrémités opposées de la came 37 sont engagées dans des ouvertures 50 diamétralement opposées du tube 35.

Dans sa partie centrale, la came 37 est percée d'un trou traversant 55 permettant le passage de la vis 1 et plus précisément de sa partie filetée 24. Le disque terminal 53 sert de butée axiale pour le bon positionnement de la came 37 par rapport au tube extérieur 35. En effet, il faut impérativement que le trou 55 de passage de la queue filetée 24 de la vis 1 soit centré dans l'outil de vissage 31. Le disque 53 peut être remplacé par tout autre moyen équivalent.

Le tirant 32 est muni d'un bouton manuel 57 de manoeuvre, faisant saillie à l'extérieur de la poignée 36 du tube 35.

La mise en oeuvre de l'outil qui vient d'être décrit s'effectue de la manière suivante.

On introduit la vis 1 dans le trou 42 de passage du tube 35, dans la rondelle 38 et dans le trou 55 de la came 37. Puis on visse son extrémité filetée 24 dans l'alésage taraudé 34 du tirant 32. L'outil 31 et la vis 1 se présentent alors dans la situation illustrée à la figure 7.

On fait tourner le tube 35 autour de son axe YY par la poignée 36, ce qui entraîne également en rotation la came 37 autour de l'axe YY. Cette rotation provoque par les faces opposées de la surface 51, des poussées axiales opposées sur l'extrémité 45 du tube 46 et sur la rondelle 38. Le tube 46 étant bloqué en translation sur le tirant 32, seule la rondelle 38 est déplacée et appliquée sur la partie 44 de la vis, en coulissant sur les goujons 47. Une fois la rondelle 38 fortement serrée sur la partie 44, le vissage ou le dévissage de la vis 1 peut être effectué.

En variante le tirant intérieur 32 peut présenter, comme dans la réalisation précédente, un alésage intérieur taraudé dont la longueur est adaptée à celle de la partie filetée de l'implant 1, par exemple un alésage taraudé long correspondant à la tige 3 d'une vis 1.

Il convient de noter que chacun des tirants 5 et 32 peut avoir ses deux extrémités opposées pourvues de trous taraudés 6, 23 de largeurs différentes, adaptées à des tiges filetées de longueurs correspondante. Leurs boutons de manoeuvre 15, 57 sont alors fixés sur l'extrémité non utilisée pour la vis choisie.

Les deux outils qui viennent d'être décrits sont utilisables dans tous les domaines techniques nécessitant le vissage d'une vis à deux tiges filetées de pas différents et réunies par une partie intermédiaire de vissage.

## Revendications

1. Outil de vissage d'une vis (1) à deux parties filetées (3, 24) l'une ayant un filet d'ancrage osseux et l'autre un filet mécanique, séparées par une partie intermédiaire (4) prévue pour recevoir des moyens de vissage, caractérisé en ce qu'il comprend :
- un tirant (5; 32) à extrémité taraudée adaptée pour venir se visser sur l'une (3; 24) des deux parties filetées,
- une cage tubulaire (7) disposée dans le prolongement de l'extrémité du tirant et en contact avec celle-ci, cette cage étant adaptée pour pouvoir coiffer ladite partie intermédiaire de vissage et étant percée d'au moins une ouverture (8) contenant un organe de roulement (9) pouvant être appliqué sur la surface de ladite partie intermédiaire par application d'une sollicitation extérieure radiale (F) sur ledit organe de roulement,
- un tube (11) enveloppant la cage et le tirant, pourvu de moyens (12) d'entraînement en rotation autour de son axe longitudinal (XX), dont la paroi intérieure est en contact avec ledit organe de roulement et profilée (14) pour qu'une rotation du tube déclenche ladite sollicitation radiale (F) et déplace radialement l'organe de roulement à travers la cage et le place en appui de serrage contre ladite partie intermédiaire (4) de la vis (1).

2. Outil selon la revendication 1, caractérisé en ce qu'il comporte plusieurs organes de roulement constitués par des galets (9) disposés avec jeu dans des ouvertures (8) formées à intervalles angulaires réguliers dans la cage (7).

3. Outil selon la revendication 2, caractérisé en ce que la paroi intérieure du tube (11) présente en regard de chaque galet, une empreinte circulaire (14) de rayon supérieur au rayon de la paroi cylindrique (15) entre ces empreintes.

4. Outil selon la revendication 1, 2 ou 3, caractérisé en ce que la cage (7) est montée dans l'extrémité du tube (11) de manière amovible.

5. Outil selon la revendication 4, caractérisé en ce que la cage (7) est pourvue de moyens de clipsage à l'intérieur de l'extrémité (13) du tube (11). par exemple au moins une languette longitudinale flexible (17) dont l'extrémité est pourvue d'un bossage (19) venant se loger dans une gorge intérieure (22) de l'extrémité (13) du tube extérieur (11).

6. Outil selon l'une des revendications 1 à 5, caractérisé en ce que le tirant (5) présente à ses extrémités opposées deux alésages taraudés (6; 23) de longueurs différentes, adaptés à des vis (1) dont les parties à filet mécanique (3; 24) destinées à être vissées dans le tirant ont des longueurs différentes.

7. Outil selon l'une des revendications 1 à 6, caractérisé en ce que le tube (11) est muni d'une poignée manuelle (12) d'entraînement en rotation traversée axialement par l'extrémité du tirant (5), lequel est dimensionné longitudinalement de manière que lorsque son extrémité opposée est en contact avec la cage (7), et qu'un jeu (j) de fonctionnement reste réservé entre la poignée (12) d'entraînement du tube et l'extrémité correspondante (15) du tirant.

8. Outil selon la revendication 7, caractérisé en ce que l'extrémité du tirant (5) traversant la poignée (12) est un bouton manuel (15) de commande placé dans un logement (16) de la poignée du tube, le jeu précité (j) étant délimité entre ce bouton et le fond (16a) du logement (16).

9. Outil de vissage d'une vis (1) à deux parties filetées (2, 3; 2, 24) séparées par une partie intermédiaire (4) prévue pour recevoir des moyens de vissage, caractérisé en ce qu'il comprend :
- un tirant (32) à extrémité taraudée adaptée pour venir se visser sur l'une (24, 3) des deux parties filetées (3; 24),
- un tube extérieur (35), contenant le tirant (32) et dans une extrémité (33) duquel la vis peut être partiellement introduite pour que l'une (24) de ses parties filetées puisse être vissée dans le tirant, ce tube étant équipé de moyens (36) d'entraînement en rotation autour de son axe longitudinal (YY),
- une came transversale (37) s'étendant diamétralement à travers ladite extrémité du tube adaptée pour recevoir la vis, et solidaire du tube en rotation,
- un premier élément (38) de serrage de la vis, interposé dans le tube avec jeu entre la came et l'extrémité adjacente (39) du tube (55), profilé pour pouvoir être déplacé en translation axiale et appliqué sur ladite partie intermédiaire (44) de la vis (1) par rotation du tube et de la came autour de l'axe longitudinal du tube.

10. Outil selon la revendication 9, caractérisé en ce que la came (37) est une barrette présentant une surface profilée (51) complémentaire de la surface (49) dudit premier élément de serrage (38) de manière qu'une rotation de la came autour de l'axe (YY) du tube (35) provoque une translation dudit élément, l'écartant de la came, et cette barrette est percée d'une ouverture centrale (55) de passage de la partie à filet mécanique (24) de la vis (1).

11. Outil selon la revendication 10, caractérisé en ce que la came (37) présente une section transversale sensiblement ovale ou ellipsoïde et est pourvue de moyens de fixation au tube (35).

12. Outil selon la revendication 11, caractérisé en ce que lesdits moyens de fixation comprennent un trou (54) formé dans la came (37) et adapté pour recevoir un goujon.

13. Outil selon la revendication 10, 11 ou 12, caractérisé en ce qu'il comprend un second élément (45), interposé entre la came (37) et le tirant (32), et qui est relié au premier élément (38) par des moyens (47) laissant audit premier élément un degré de liberté en translation axiale dans le tube.

14. Outil selon la revendication 13, caractérisé en ce que ledit premier élément est une rondelle (38) présentant une surface interne (49) adaptée pour coiffer la surface de la partie intermédiaire (44) de la vis (1), et ledit second élément (45) est prolongé par un tube longitudinal intermédiaire (46) interposé entre le tube extérieur (35) et le tirant (32) et contenant ce dernier, et ce tube intermédiaire est relié à la rondelle (38) par des goujons (47) permettant une libre translation de la rondelle.

15. Outil selon la revendication 9, caractérisé en ce que le tirant (32) est pourvu de deux extrémités taraudées dont les trous (34) ont des longueurs différentes, correspondant à des longueurs différentes des parties filetées (3; 24) associées d'une vis (1).
